(19) Europäisches Patentamt
European Patent Office
Office européen des brevets

(11) **EP 4 645 218 A1**

(12) **EUROPEAN PATENT APPLICATION**

(43) Date of publication:
**05.11.2025 Bulletin 2025/45**

(21) Application number: **24173486.2**

(22) Date of filing: **30.04.2024**

(51) International Patent Classification (IPC):
***G06T 7/00*** *(2017.01)* ***G06T 7/11*** *(2017.01)*

(52) Cooperative Patent Classification (CPC):
**G06T 7/11; G06T 7/194; G06T 15/08;**
G06T 2207/10081; G06T 2207/20084;
G06T 2207/30008; G06T 2210/41; G06V 2201/031

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Siemens Healthineers AG**
**91301 Forchheim (DE)**

(72) Inventors:
• **Engel, Klaus**
**90411 Nürnberg (DE)**
• **Datar, Manasi**
**411038 Pune (IN)**
• **Ghesu, Florin-Cristian**
**91083 Baiersdorf (DE)**
• **Teichmann, Marvin**
**91054 Erlangen (DE)**

(74) Representative: **Siemens Healthineers**
**Patent Attorneys**
**Postfach 22 16 34**
**80506 München (DE)**

Remarks:
Amended claims in accordance with Rule 137(2) EPC.

(54) **A COMPUTER-IMPLEMENTED METHOD FOR RENDERING A VOLUMETRIC DATASET REPRESENTING A MEDICAL VOLUME**

(57) A computer-implemented method for rendering a volumetric dataset representing a medical volume is described. The method comprises: performing a segmentation process on a volumetric dataset to determine segmentation data indicative of an association between a given location in the volumetric dataset and a given one of one or more anatomical regions in the medical volume, and performing an uncertainty determination process on the segmentation data to determine uncertainty data indicative of an uncertainty in the association between the given location and the given one of the one or more anatomical regions. The method also comprises performing a volume rendering process on the volumetric dataset, using the uncertainty data, to render an image of the medical volume, including determining a visual characteristic of the image based on the uncertainty in the association between the given location and the given one of the one or more anatomical regions.

Fig 1

**Description**

Technical Field

The present invention relates to a computer-implemented method for rendering a volumetric dataset representing a medical volume. The present invention also relates to a set of machine-readable instructions for implementing the method, a machine-readable medium comprising such instructions and an apparatus for implementing the method.

Background

In computer-implemented imaging of medical volume data, e.g., CT scan data, segmentation algorithms may be used to produce segmentation results identifying anatomical regions, e.g. organs, to which locations in the medical volume data correspond. Such segmentation algorithms are often AI-driven and may also be referred to as auto-contouring algorithms.

One example context in which such segmentation algorithms may be applied is radiation therapy (RT) planning. In RT planning, a segmentation algorithm may be used to provide a segmentation of a volumetric dataset indicating organs or other anatomical regions which should be spared from radiation, which may be also referred to organs at risk (OAR). The results of the segmentation may be presented to a human expert, such as a dosimetrist or radiation oncologist, to be verified and, if necessary, edited. Such verification may involve significant time and effort on the part of the human expert. For example, recent auto-contouring algorithms for RT planning produce 153 contours of organs/anatomies which should be spared from radiation, each of which may need to be verified by a human expert.

Summary

According to a first aspect of the present invention, there is provided a computer-implemented method for rendering a volumetric dataset representing a medical volume, the method comprising: performing a segmentation process on a volumetric dataset to determine segmentation data indicative of an association between a given location in the volumetric dataset and a given one of one or more anatomical regions in the medical volume; performing an uncertainty determination process on the segmentation data to determine uncertainty data indicative of an uncertainty in the association between the given location and the given one of the one or more anatomical regions; and performing a volume rendering process on the volumetric dataset, using the uncertainty data, to render an image of the medical volume, including determining a visual characteristic of the image based on the uncertainty in the association between the given location and the given one of the one or more anatomical regions.

The volume rendering process may comprise: determining visual parameter data for a given position in the image based on one or more respective visual parameter values for one or more sample points in the volumetric dataset, and wherein the visual parameter value for a given sample point of the one or more sample points is determined based on: an uncertainty value at the given sample point derived from the uncertainty data.

The visual parameter value for the given sample point may be determined based on one or more of: a volume value at the given sample point derived from the volumetric dataset; and a segmentation value at the given sample point derived from the segmentation data.

The visual parameter value for the given sample point may be determined by applying one or more transfer functions to one or more of: the uncertainty value at the given sample point; the or a volume value at the given sample point; and the or a segmentation value at the given sample point.

The visual parameter value for the given sample point may be determined by combining: a color and/or an opacity value obtained by applying an uncertainty transfer function to the uncertainty value; with one or more respective color and/or opacity values obtained by applying respective transfer functions to one or more of the volume value at the given sample point and the segmentation value at the given sample point.

The volume rendering process may be a physically based volume rendering process.

The segmentation process may comprise applying a trained machine-learning model to the volumetric dataset and obtaining the segmentation data as an output of the trained machine-learning model.

The uncertainty determination process may comprise determining the uncertainty data by performing a statistical calculation on the segmentation data.

For the given location in the volumetric dataset: the segmentation data may comprise first data including one or more respective probability values indicative of respective likelihoods that the given location corresponds to each of the one or more anatomical regions; the uncertainty data may comprise second data including one or more uncertainty values indicative of respective uncertainties in the respective probability values of the first data.

The segmentation process may comprise determining the association between the given location and the given one of the one or more anatomical regions based on the respective probability values of the first data.

The uncertainty determination process comprises determining the uncertainty in the association between the given location and the given one of the one or more anatomical regions based on the uncertainty values of the second data.

Determining the uncertainty in the association between the given location and the given one of the one or more anatomical regions based on the uncertainty values of the second data may comprise determining the largest of the uncertainty values of the second data.

The method may furthermore comprises utilizing the image of the medical volume in a radiation therapy planning process.

According to a second aspect of the present invention, there is provided a computer-implemented method for representing an uncertainty in a segmentation of a volumetric dataset representing a medical volume, the method comprising: performing a segmentation process on a volumetric dataset to determine segmentation data indicative of an association between a given location in the volumetric dataset and a given one of one or more anatomical regions in the medical volume; performing an uncertainty determination process on the segmentation data to determine uncertainty data indicative of an uncertainty in the association between the given location and the given one of the one or more anatomical regions; performing a numerical analysis process on the uncertainty data to obtain one or more numerical analysis results; and presenting the one or more numerical analysis results to a user.

Presenting the one or more numerical analysis results to a user may comprise presenting a graphical representation of the one or more numerical analysis results to the user.

According to a third aspect of the present invention, there is provided a set of machine-readable instructions which when executed by a processor cause a method according to the first aspect or the second aspect to be performed.

According to a fourth aspect of the present invention, there is provided a machine-readable medium comprising a set of machine-readable instructions according to the third aspect.

According to a fifth aspect of the present invention, there is provided an apparatus comprising a processor and a storage comprising a set of machine-readable instructions according to the fourth aspect.

According to a further aspect of the present invention, there is provided a computer program or a computer-readable medium comprising instructions which, when executed by a computer, cause the computer to carry out the method according to the first aspect or the second aspect.

According to a further aspect of the present invention, there is provided an apparatus comprising a processor configured to perform the method according to the first aspect or the second aspect.

Brief Description of the Drawings

The present invention will now be described, by way of example only, with reference to the following figures, in which:

Figure 1 is a flow chart illustrating a computer-implemented method for rendering a volumetric dataset representing a medical volume;

Figure 2 is a schematic drawing illustrating aspects of a volume rendering process of an example method of rendering the volumetric dataset;

Figures 3 to 5 show rendered images of medical volume data, each of the images having been produced by an example method described herein;

Figure 6 is a flow chart illustrating a computer-implemented method for representing an uncertainty in a segmentation of a volumetric dataset representing a medical volume; and

Figure 7 shows an apparatus for performing example methods described herein.

Detailed Description

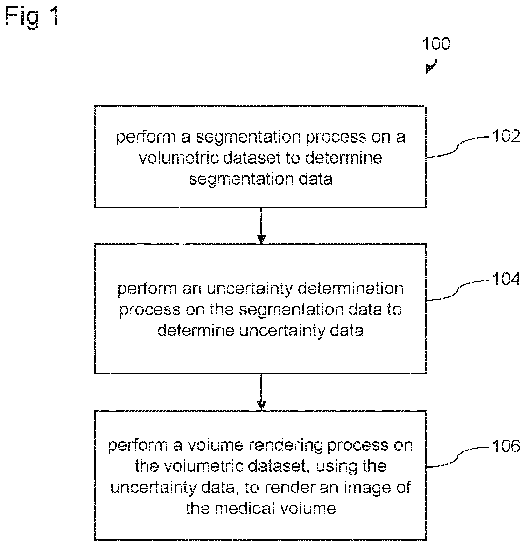

Figure 1 is a flow chart illustrating an example computer-implemented method 100 for use in rendering a volumetric dataset representing a medical volume.

The volumetric dataset may comprise a discrete sampling of a scalar field. The volumetric dataset may, for example, be obtained by loading from a memory, sensors, and/or other sources. The medical volume represented by the volumetric dataset may, for example, represent a patient or a part of a patient, for example a human or animal patient. In some examples described herein, the medical volume includes a pelvic region of a human patient.

In general, any suitable scanning modality may be used to produce the volumetric dataset. For example, the scanning modality may comprise the use of computed tomography (CT), or of magnetic resonance imaging (MRI). In some examples a scanning modality comprising the use of positron emission tomography (PET), single photon emission computed tomography (SPECT), ultrasound, or another scan modality may be used. Scan data may be provided in the form of multiple two-dimensional (2D) scans or may be formatted from a scan. In some examples, the volumetric dataset may comprise a stack of 2D image slices. The 2D image slices may comprise compressed slice data, for example, in JPEG2000 format. In some examples, the volumetric dataset is a DICOM dataset created by scanning at least a portion of a patient using a scanning modality.

The volumetric dataset may comprise data formatted as a plurality of voxels. The voxels may, for example, be in a uniform or non-uniform grid, or may be arranged in some other type of geometry (e.g., polar coordinate format). The voxels may be isotropic or anisotropic. Each voxel may typically represent a scalar value obtained by sampling a scalar field, although in some examples the volumetric dataset may comprise data relating to a non-scalar field. The type of value represented by each voxel may be dependent on the means by which the volumetric dataset is obtained. For example, where a CT scanner is used to produce the volumetric dataset, the dataset may comprise Hounsfield values. The values represented by each voxel may be represented at a given precision, for example, 8-bit or 16-bit.

At block 102, the method 100 comprises performing a segmentation process on the volumetric dataset to determine segmentation data. The segmentation data is indicative of an association between a given location in the volumetric dataset and a given one of one or more anatomical regions in the medical volume.

For example, the given location may be a voxel of the volumetric dataset. The segmentation data may be indicative that the voxel is associated with a particular anatomical organ or other anatomical region, for example, the heart or the liver. The segmentation process may be applied to some or all of the voxels in the volumetric dataset. The segmentation data may indicate for each voxel in the volumetric dataset to which the segmentation process is applied an association between the given location and the one of the one or more anatomical regions in the medical volume.

The segmentation process may be configured to determine whether a given voxel is associated with one of a discrete number of anatomical regions of interest. The anatomical regions of interest may, for example, depend on the clinical context for which the visualization is being generated. For example, in an example where the volumetric dataset represents a pelvic region of a patient, the segmentation process may be configured to determine, for each given voxel, first data indicative of whether the voxel is associated with one of a set of organs at risk or is not associated with any of the organs at risk. In one simplified example, the organs at risk may be, the bladder, the left femur, the right femur. The segmentation algorithm may then determine data indicative of whether each voxel is associated with the bladder, the left femur, the right femur, or none of these. For example, the uncertainty data may indicate that a first voxel is associated with the bladder, a second voxel is associated with the left femur, and a third voxel is not associated with any of the left femur, right femur or bladder.

In some examples, the segmentation data produced by the segmentation process may comprise, for each given location, first data including respective values indicating a probability that the given location corresponds to each of the plurality of anatomical regions of interest. The first data may comprise a vector or array comprising these values. For example, returning to the case where the medical volume is a pelvic region, the segmentation process may be configured to determine, for a given voxel, first data comprising respective probability values corresponding to each of: the bladder, the left femur, the right femur and none of these. In such an example, the first data for each voxel may comprise a vector or array of length four containing the four probability values *(P(bladder), P(left femur), P(right femur), P(none))*.

In some examples, the segmentation process may comprise determining, based on the first data, the anatomical region with which the given voxel is associated. For example, the values of the first data which indicate the respective likelihoods that the voxel corresponds with each of the anatomical regions may be thresholded to determine an association between the voxel and the given one of the anatomical regions of interest. For example, the anatomical region with the highest determined probability for the given voxel may be determined as the anatomical region with which the voxel is associated. For example, where the determined probabilities for a given voxel are *(P(bladder), P(left femur), P(right femur), P(none)) = (0.95, 0.00, 0.00,* 0.05), the highest of the determined probabilities (0.95) is the probability associated with the bladder. The voxel may therefore be determined to be associated with the bladder.

In some examples, each voxel is labelled according to the anatomical region with which it is determined to be associated. A segmentation volume comprising the segmentation data including the labels for each voxel may thereby be generated. The segmentation volume may be in registration with the volumetric dataset and may be used during a volume rendering process to allow the segmentation data to be visualized, as will be described in examples below.

At block 104, the method 100 comprises performing an uncertainty determination process on the segmentation data to determine uncertainty data. The uncertainty data is indicative of an uncertainty in the association between the given location and the given one of the one or more anatomical regions.

In some examples, the uncertainty for a given voxel is a measure of an uncertainty in the classification of the voxel by a machine-learning model. In some examples, the uncertainty in the segmentation may derive from the fact that the medical volume is one which represents a case unfamiliar to the segmentation algorithm. For example, the segmentation algorithm may be an AI-driven algorithm which has been trained on medical scan training data. The medical scan training data may comprise many different examples of medical scans. However, in clinical practice, a volumetric dataset to which the trained model is applied may be one which is not represented or is only minimally represented in the training data. For example, the medical volume may include an object, such as a medical implant, e.g. a brachytherapy implant, which was not present in examples of medical volumes included in the training data. If the trained model is applied to such a case, then the segmentation results may be highly uncertain. This may be referred to as epistemic uncertainty. Other sources of uncertainty in the segmentation result may be what is referred to as aleatoric uncertainty. For example, noise or other sources of randomness in the volume data may make the result of the segmentation algorithm uncertain for a given voxel.

The uncertainty data may be stored in association with the volumetric dataset to be used in generating an image, as will be discussed in more detail below.

In some examples, the uncertainty data comprises, for each voxel, a value indicating an uncertainty in the segmentation result for that voxel.

In some examples, where the segmentation data comprises first data including values indicating respective probabilities of an association between the given voxel and the one or more anatomical regions of interest, the uncertainty data may comprise, for each given voxel, second data including values indicating a measure of uncertainty in each of these probabilities. In some such cases, an overall uncertainty for the voxel may be determined based on the multiple uncertainty values for the voxel. For example, the largest of the uncertainty values for the given voxel may be taken as the overall uncertainty for the voxel. For example, in the above example where the first data indicates *(P(bladder), P(left femur), P(right femur), P(none)) = (0.95, 0.00, 0.00,* 0.05), the second data may indicate respective uncertainty values providing a measure of an uncertainty in each of these probabilities, for example *uncertainty(bladder, left femur, right femur, none) = (0.0007, 0.0000, 0.0000, 0.0001).* The overall uncertainty for the voxel may be determined as the maximum of the respective uncertainty values for the anatomical regions, that is, 0.0007.

In another example, the overall uncertainty value for each voxel may be the uncertainty value associated with a particular one of the anatomical regions of interest, for example, the bladder. This may be useful where it is desired to visualize the segmentation result and the associated uncertainty in that result for a particular anatomical organ of interest, as will be discussed in more detail below.

Where the segmentation process involves the application of a machine-learning model, the measure of uncertainty may be an output of the machine-learning model. For example, the machine-learning model may generate multiple estimates of the respective probabilities that each given voxel relates to each of one or more anatomical regions. These multiple estimates may be obtained, for example, via an MC dropout process. In another example, multiple estimates may be obtained from respective machine-learning models forming an ensemble. The uncertainty may be derived as a statistical property, such as a variance or standard deviation, of these multiple estimates. Further, the uncertainty data may also be derived from the multiple estimates. For example, an average, e.g. a mean, of the multiple estimates may be determined to provide the values of the first data for a given voxel.

As an example, the machine-learning model may provide first, second and third estimates of the probabilities that a given voxel is associated with each of the bladder, left femur, right femur and none of these as follows:

probability_estimate1 = $(P_1(bladder), P_1(left\ femur), P_1(right\ femur), P_1(none)) = (0.92, 0.00, 0.00, 0.04)$;
probability_estimate2 = $(P_2(bladder), P_2(left\ femur), P_2(right\ femur), P_2(none)) = (0.95, 0.00, 0.00, 0.06)$;
probability_estimate3 = $(P_3(bladder), P_3(left\ femur), P_3(right\ femur), P_3(none)) = (0.97, 0.00, 0.00, 0.05)$.

The first data, comprising the respective probabilities that the voxel corresponds to the different anatomical regions, may be computed from the estimates as:
mean(probability_estimate1, probability_estimate2, probability_estimate3) = (0.95, 0.00, 0.00, 0.05).

In this example, the first data may be considered indicative that the voxel is associated with the bladder since the maximum probability is that associated with the bladder.

The second data for the voxel may be computed from the estimates as:
uncertainty(P(bladder), P(left femur), P(right femur), P(none)) = variance(probability_estimate1, probability_estimate2, probability_estimate3) = (0.0007, 0.0000, 0.0000, 0.0001).

In this example, the overall uncertainty for the voxel may be determined as 0.0007, on the basis that this is the largest of the computed variances for the voxel.

The uncertainty determination process may result in uncertainty data being generated for each of the voxels in the volumetric dataset to which the segmentation process and uncertainty determination process is applied. This may be all or some of the voxels in the volumetric dataset. Accordingly, similarly to as described above for the segmentation data forming a segmentation volume, an uncertainty volume comprising the uncertainty data may be formed.

The uncertainty volume may, for example, comprise the determined overall uncertainty values for each voxel. The uncertainty volume may be in registration with the volumetric dataset. The segmentation volume may be used during a volume rendering process to allow the uncertainty data to be visualized.

At block 106, the method 100 comprises performing a volume rendering process on the volumetric dataset, using the uncertainty data, to render an image of the medical volume, including determining a visual characteristic of the image based on the uncertainty in the association between the given location and the given one of the one or more anatomical regions.

The volume rendering process may be a physically based rendering process, for example, a path tracing process. Determining the visual characteristic of the image may, for example, comprise determining one or more visual parameter values of the volume rendering process, such as color and/or opacity values of one or more sample points of a path tracing process.

The image of the medical volume created by performing the rendering process can be utilized in a radiation therapy planning process. In particular, the image of the medical volume can be displayed in a software configured for radiation therapy planning. In particular, instructions can be provided to the software to focus the display of the image of the medical volume at certain regions with high uncertainty.

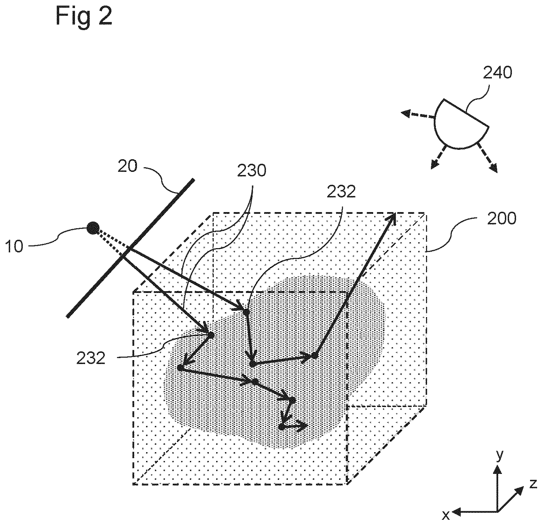

Referring now to Figure 2, there is illustrated schematically an example of the volume rendering process performed on a volumetric dataset 200. The volumetric dataset 200 in this example has cuboidal geometry. In the example of Figure 2, the first physically based rendering process is a path tracing process.

In the path tracing process represented by Figure 2, a viewpoint 10 is defined with respect to the volumetric dataset 200. A viewing plane 20 is located in front of the viewpoint 10. The viewing plane 20 comprises a plurality of image positions, for example, pixels (not shown in Figure 2), and allows construction of a visualization of the volumetric dataset 200, as viewed from the viewpoint 10. The pixels may, for example, be arranged in a grid in the viewing plane 20.

To obtain visual parameter data to be used to render a visualization of the volumetric dataset 200 as viewed from the viewpoint 10, a plurality of simulated paths 230 through the volumetric dataset 200 are determined. Each of the paths may be used to determine a contribution to a visual parameter value for a given pixel in the viewing plane 20. The visual parameter value may, for example, include a color value for the given pixel.

Path tracing simulates light propagation through the volumetric dataset 200, including simulating scattering and absorption, by tracing a plurality, for example millions, of simulated light paths 230. Each of the paths 230 may be used to generate a contribution to a visual parameter value for a given pixel in the viewing plane 20. The contributions may be used to determine an estimate of the light hitting the given pixel. The contribution for a given path 230 may be determined by sampling, at a plurality of sampling points along the given path 230, the data forming the volumetric dataset 200. The sampling points along the given path 230 may, for example, be substantially equidistantly spaced along the path 230. By averaging many such estimates obtained from many simulated paths 230 for a given pixel in the viewing plane 20, visual parameter data may be obtained for the pixel. By repeating this process for each of the pixels in the viewing plane 20, visual parameter data may be accumulated for allowing an image from the perspective of the viewpoint 10 to be rendered on the viewing plane 20.

According to an example, for a given path 230 associated with a given pixel in the viewing plane 20, to determine an initial direction of the path 230, a pseudo-random position in the given pixel may be determined. The initial direction for the path 230 may then be generated by connecting the pseudo-random position in the given pixel with a pseudo-random position on a simulated camera lens (not shown) at the viewpoint 10. The intersection of the path 230 with a bounding box of the volumetric dataset 200 is then computed. The path 230 is then traced through the volumetric dataset 200 and sampled in the manner described above.

Scatter events along the path 230 are simulated, for example, by Woodcock tracking or, for example, by accumulating opacity along the path 230 and triggering a scatter event when the opacity exceeds a scatter event threshold. When a scatter event occurs along the path 230, the path 230 is continued by determining a new ray originating at a scattering position 232. The new ray following a first scatter event along a given path 230 may be referred to as the secondary ray, while the ray prior to the first scatter event along the given path 230 is referred to as the primary ray. In Figure 2, scattering positions are represented by a solid dot along the paths 230. For clarity, for each of the paths 230, only a first scattering position 232 is labelled. A direction of the new ray may, for example, be determined using a phase function, that is, a probability density function for the scattering direction, at the scattering position 232. The phase function may, for example, be determined based on a sampled volume value (for example, where the volumetric dataset 200 is produced by CT scanning, a Hounsfield value) at the scattering position 232.

The new ray is traced in a similar manner to the initial ray to determine the next scattering position, if any, for the path 230. The same operations are performed at the next scattering position as at the first scattering position 232. This path tracing process continues until a ray of the path 230 leaves or is absorbed by the volumetric dataset 200. Absorption by the volumetric dataset 200 can be modelled, for example, by using a maximum threshold for the number of scattering events, or probabilistically by using an extinction probability density function based on the number of scattering events. In one example, a Russian Roulette algorithm may govern extinction of the path 230 by, pseudo-randomly determining at each scattering position whether to continue or to terminate the path 230. In yet another example, an accumulated opacity may be determined along the path 230 and the path 230 may be terminated when the accumulated opacity reaches a probabilistic threshold, computed from a probability density function (PDF). If the path 230 leaves the volumetric dataset 200 before being terminated, a visual parameter value contribution for the path 230 may be determined. On the other hand, if the path 230 is terminated before leaving the volumetric dataset 200, no visual parameter data value contribution is computed, and the path 230 makes no contribution to the visual parameter data of the pixel to which it corresponds.

A sampling process is performed at a plurality of sampling points (not shown) along the given path 230. At a given sampling point along the path 230, the sampling process provides a volume value for the sampling point. The volume value may be used, as described below, to obtain visual parameter data for the sampling point, for example, by use of a transfer function. The sampling process may comprise interpolating a volume value for the sampling point from the first volume data and/or the second volume data, for example, using trilinear interpolation.

The sampling process may also comprise sampling the uncertainty data to obtain an uncertainty value for the sampling point. The sampling process may also comprise sampling the segmentation data to obtain a segmentation value for the given sampling point. The segmentation value for the sampling point and the uncertainty value for the sampling point may be obtained by sampling the segmentation volume and uncertainty volume described above in relation to blocks 102 and 104 of method 100. Any suitable sampling method may be used in either case. For example, the segmentation value at the sampling point may be interpolated in any suitable manner from the segmentation volume. Similarly, the uncertainty value at the sampling point may be interpolated in any suitable manner from the uncertainty volume.

For a given sampling point along the path 230, once the volume value, the segmentation value and the uncertainty value for the sampling point have been obtained, a classification process may be performed to obtain visual parameter data for the sampling point based on volume value, the segmentation value and the uncertainty value. The visual parameter data may, for example, comprise a color and/or an opacity. The visual parameter data may comprise both a color and an opacity, for example, an RGBA value.

Performing the classification process may comprise applying respective transfer functions to the volume value, the segmentation value and the uncertainty value. The application of each transfer function to the relevant value may provide a color and/or an opacity value. These color and/or opacity values may be combined, for example, by summing, blending or multiplying, to provide an overall sample color and/or opacity for the sampling point.

For example, a volume transfer function may be applied to the volume value to obtain a volume color for the sampling point. A segmentation transfer function may be applied to the segmentation value to obtain a segmentation color at the sampling point. An uncertainty transfer function may be applied to the uncertainty value to obtain an uncertainty color at the sampling point. In each case, the application of the transfer function may comprise using the relevant value to look up a color value from a respective color map. Similarly, each transfer function may provide a respective opacity, which may also be referred to as an alpha value, for the sampling point by performing a look-up using the relevant value. These opacity values may be combined in a similar way to the color values to obtain an overall opacity value for the sampling point.

In other words, a sample color *Csample* at a sampling point p in the volumes V (anatomical volume *anatomy,* segmentation volume seg, uncertainty volume *unc*) and color palettes anatomy volume palette *Panatomy,* segmentation volume palette *Pseg,* uncertainty volume palette *Punc*) may be computed as:

$$Csample \; = \; Panatomy\big(Vanatomy(p)\big) + \; Pseg\big(Vseg(p)\big) + \; Punc\big(Vunc(p)\big)$$

or any other mathematical combination of the color components (for example, over blending, mixing, multiplication, etc.)

For each transfer function, the color map may be window-levelled, that is, mapped to an appropriate, for example, a pre-defined or user-controlled, range of values to provide the desired mapping of colors to values. For example, uncertainty colors may be thresholded to filter only high uncertainty values and contribute uncertainty color palette values only in areas of high uncertainty. This allows for locations with high uncertainty to be highlighted in the visualization. The color palettes, for example, the uncertainty color palette, may contain high dynamic range colors. This can be used to achieve effects such as a glowing appearance in areas of high uncertainty.

In examples, a rendering algorithm may model an illumination effect by modelling a light source 240 illuminating the volumetric dataset 200. The illumination effect may be taken into account when determining opacity and color values at the sampling points. For example, a shading computation may be applied at the sampling point, taking into account simulated light at the sampling point. The light source 240 may be a point source, a directional light source, or may comprise a light map. In some examples, the light source may comprise a high-definition light map. The light map may, for example, when the volumetric dataset 200 is cuboidal, have six sides corresponding to outer sides of the volumetric dataset 200. In some examples, the simulated light source 240 may be a model of a light-emitting object, or a combination of models of multiple, for example, different, light sources. In some examples, parts of the volumetric dataset 200 itself may emit light.

The process of obtaining visual parameter data may be performed for each of the sampling points along the path 230. The visual parameter data gathered at each of the sampling points along the path 230 may be used to generate the visual parameter data contribution for the path 230. For example, visual parameter data obtained at each of the sampling points may be accumulated along the path 230 to provide the visual parameter data contribution for the path 230.

The visual parameter data contribution for the path 230 may then be stored in association with the pixel to which the path 230 corresponds. The visual parameter data contribution may be stored in a buffer, for example, a high dynamic range (HDR) buffer. The procedure may be repeated many times for the given pixel by tracing many paths to obtain many corresponding visual parameter data contributions for the given pixel.

The procedure of obtaining visual parameter data contributions from a plurality of paths may be repeated for each of the pixels in the viewing plane 20, resulting in visual parameter data being stored in the buffer in association with each pixel in the viewing plane 20.

To render the volumetric dataset, a tone-mapping pass may be applied to the visual parameter data stored in the buffer. The visual parameter stored in the buffer for a given pixel may, as described above, comprise a plurality of visual parameter data contributions, each contribution having been generated by tracing a particular path 230 associated with the given pixel. The visual parameter data contributions stored in the buffer for a given pixel may be averaged or otherwise used to generate overall visual parameter data values for the given pixel. The tone-mapping pass may be performed repeatedly as the number of paths 230 traced and stored in the buffer increases. Accordingly, the image may be progressively refined as the number of paths 230 traced increases.

Example methods described herein may allow for uncertainty data representing the uncertainty in the results of a segmentation process to be viewed by a user in an effective and efficient manner. Since the uncertainty data is used as part of the volume rendering process, an integrated visualization of the uncertainty data and the medical volume may be provided. Since this visualization is a volume rendering process, the segmentation result and its associated uncertainty is presented to the user in the appropriate 3D anatomical context. This may allow the user to more easily and quickly identify whether the segmentation result is correct or should be edited. Further, since an integrated visualization is generated for presenting to the user, the user may be presented with all of the information required to understand and verify the segmentation result in a single view, without, for example, having to review multiple disjointed pieces of information.

Example methods further allow a user to quickly identify and focus their attention on the areas of the segmentation result which are determined to be uncertain. For example, in many cases, the segmentation result may be clinically usable without manual editing. By providing a clear visualization of the uncertainty, the method may, for example, allow for a reviewer to quickly understand when and where the segmentation result is uncertain and to approve or ignore results where the uncertainty is very low and to concentrate efforts efficiently on results which have a high degree of uncertainty. This may result in significant savings in time and effort on behalf of the user. Moreover, since the user is able to easily identify the uncertain areas, the accuracy of their review may be improved since the areas of largest uncertainty are made clear and can be made the object of their focus during the review.

By improving the review process for the results of a segmentation algorithm, the segmented visualization can be used effectively for its desired purpose, for example, for RT planning. Uncertainty visualization can also support downstream a more informed treatment planning. For example, areas affected by aleatoric uncertainty can be either spared radiation with higher tolerance, if part of an organ at risk, or irradiated with higher margin, if part of the target. The uncertainty data may therefore be used to guide the treatment plan optimization process, which may be either AI-driven or conventional. Similarly, in the context of plan review, the dose volume coverage can be assessed considering any unresolvable uncertainty in the segmentation results, i.e., the aleatoric uncertainty.

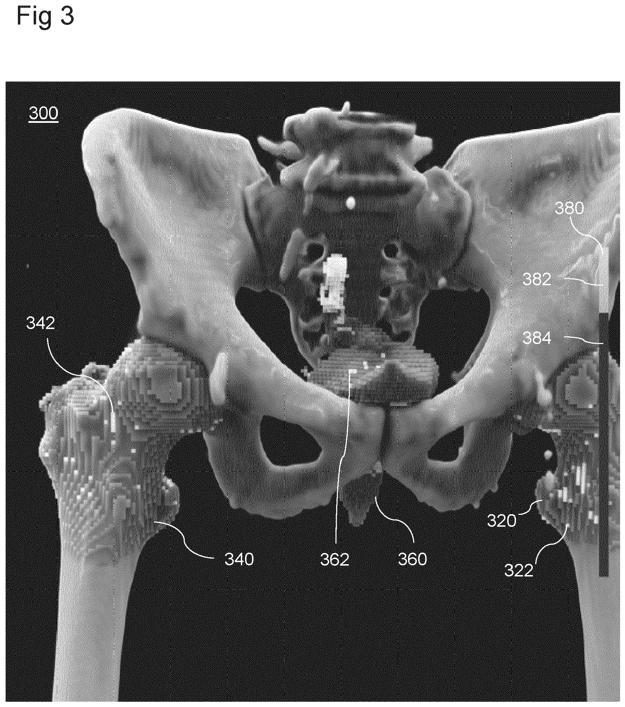

Various techniques according to methods described herein may be used to provide a desired visualization of the uncertainty data. Example images produced examples of such techniques are shown in Figures 3 to 5.

Figure 3 shows a first example image 300, rendered from a volumetric dataset representing a pelvic region of a patient. The image 300 shows a left femur 320, right femur 340, and bladder 360 each of which have been identified by a segmentation process. This image has been generated by a path tracing volume rendering process as described above with reference to Figures 1 and 2. During the sampling process, as described above with reference to Figure 2, at a given sampling point the volumetric dataset, the segmentation volume and the uncertainty volume are sampled. In this example, as described above, the segmentation data comprises, for each voxel, a respective probability that the voxel corresponds with each of a plurality of anatomical regions, including the left femur, the right femur and the bladder. A respective variance for each of these probabilities is computed and stored in association with the voxel. In this example, the uncertainty volume contains, for a given voxel, the maximum variance of the variances for that voxel.

A color map is applied to each sampled value at the sample point to obtain colors which are combined to obtain a sample color for the sample point. Volume values may be mapped to colors by any suitable transfer function. Segmentation values may also be mapped by any suitable transfer function, for example, each segmentation value may be mapped to a respective color depending on the organ which it indicates. Uncertainty values, in the example of Figure 3, are mapped to colors using a color map 380. The uncertainty color map 380 is window-levelled such that high values of uncertainty are mapped to a first color 382, which may be a high-dynamic range color, while lower values of uncertainty are mapped to a second color 384 which does not contribute to the visualization. Figure 3 shows example voxels which have a high level of uncertainty, visible due to the contribution from the first color 382, including an uncertain left femur voxel 322, an uncertain right femur voxel 342 and an uncertain bladder voxel 362.

The image shown in Figure 3 has been rendered by a block-rendering technique. In this technique, the volume value at a given sample point is smoothly interpolated from the volumetric dataset. For example, the volume value at a given sample point may be obtained using trilinear interpolation. The segmentation volume and the uncertainty volume, on the other hand, are sampled using nearest-neighbor interpolation. This provides a block-like appearance of locations in the volume which are colored according to the segmentation and/or uncertainty data, such as the regions of the image showing the left femur 320, right femur 340 and bladder 360. This block-like appearance emphasizes the analytical nature of the prediction provided by the segmentation data and the uncertainty in the prediction. This may assist a user with identifying and reviewing voxels where the segmentation result is highly uncertain.

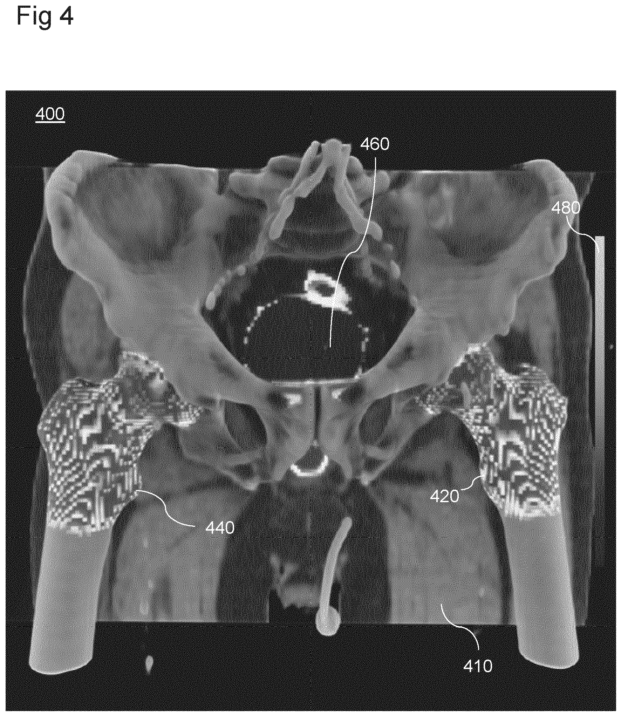

Figure 4 shows a second example image 400. The image 400 is generated by a technique involving mixing a multi-planar reconstruction (MPR) with a 3D visualization of the volumetric dataset. In this technique, an MPR plane 410 is embedded into the 3D visualization and uncertainty color values and segmentation values are shown on this plane. In Figure 4, the left femur 420, right femur 440 and bladder 460 are visible and are colored at their respective intersections between the MPR plane 410 according to a segmentation color and an uncertainty color map 480. Unlike in the example of Figure 3, the uncertainty color map 480 is not window levelled but instead provides a range of colors mapped to the entire range of uncertainty values. The MPR plane can be freely oriented and may be interactively melted through the volume to probe and inspect regions of high uncertainty. This technique may help with revealing anatomy that may be "windowed out" in a technique such as that used to generate the image shown in Figure 3.

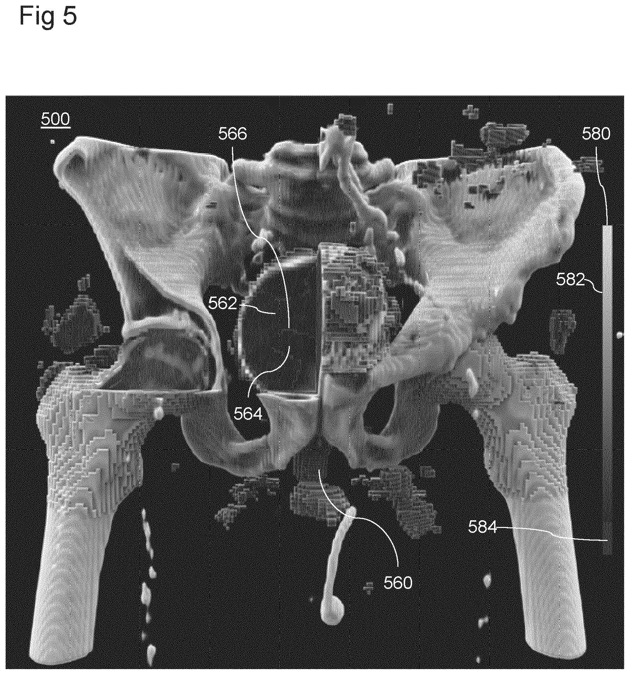

Figure 5 shows a third example image 500, generated by another example technique. In this example, clip planes have also been applied such that an interior of the bladder 560 is visible in the image 500. The image 500 is generated according to a similar technique to the image 300 described with reference to Figure 3, including the block-rendering aspects. In addition, the image illustrated by Figure 5 includes silhouettes which show the boundary between voxels which have been rendered using a color mapped from an uncertainty value and voxels which have not. In this example, uncertainty values are mapped according to the color map 580 which is window levelled and which maps uncertainties above a particular threshold to a range of colors 582 but maps very low uncertainties to a color 584 which does not contribute to the visualization. Since color values from the volume values may overlap with colors from the segmentation value and uncertainty value, in this technique, areas where the color derives from an uncertainty value are outlined. For example, in a first region 562, voxels have been identified by the segmentation process as being associated with the bladder 560 with a high degree of certainty. The voxels in the first region 562 have a segmentation color mapped from the segmentation value which indicates that they are associated with the bladder. The uncertainty values for the voxels in the first region 562 are below the window level threshold, such that they are not colored by the uncertainty color map 580. On the other hand, voxels in a second region 564 have a level of uncertainty higher than the window level threshold and therefore their uncertainty values are mapped to a color in the range 582. The overall color for voxels in this second region 564 may be obtained from a combination of the segmentation color and the uncertainty color. In the silhouette rendering technique, a silhouette or outline 566 is included in the image 500 to depict the boundary between the first region 562 where the uncertainty values are low and the second region 564 where the uncertainty values are higher. The silhouette may be rendered in any suitable way. For example, screen-space partial derivatives, e.g. d/dX and d/dY, of the uncertainty color values of neighboring pixels may be determined by the shader. A silhouette may be rendered when such a partial derivative exceeds a predefined threshold. This technique may assist a user with identifying areas where the segmentation result is uncertain, in particular, by differentiating those areas from similarly colored areas which have not been colored by the uncertainty color map.

Various other techniques not shown in the figures may be used according to example methods described herein. For example, an image may be rendered using an uncertainty density visualization rendering technique. In such a technique, with reference to Figure 2, during path tracing, uncertainty values are accumulated on the primary rays of the sampled paths 230 to obtain an accumulated uncertainty value. The accumulated uncertainty value is then mapped to a color and an opacity value. This color and opacity may then be overlayed with a 3D visualization obtained from path tracing the volumetric dataset or shown natively. This may allow for areas of high uncertainty to be easily identified.

In another example technique, a maximum uncertainty projection is used. According to this technique, the maximum uncertainty is found on primary rays during the path tracing process. The maximum variance may be mapped to a color and an opacity value and overlayed with a 3D visualization obtained from path tracing the volumetric dataset or shown natively. This may allow for areas with the highest uncertainty to be easily identified.

In some example techniques, a multi-pass volume rendering algorithm may be used. This may address the problem of areas in the volume which have high uncertainty values but which are occluded by other structures in the 3D visualization. For example, voxels with high uncertainty values may be embedded in a hull of lower uncertainty values and therefore may not be visible in some visualization techniques. In one example, a multi-pass algorithm may be used combining a technique such as an accumulated uncertainty projection or maximum variance projection, as described above, with a path tracing rendering of the volumetric dataset. This may allow for areas of high uncertainty to be visualized even when occluded by other structures in the volume.

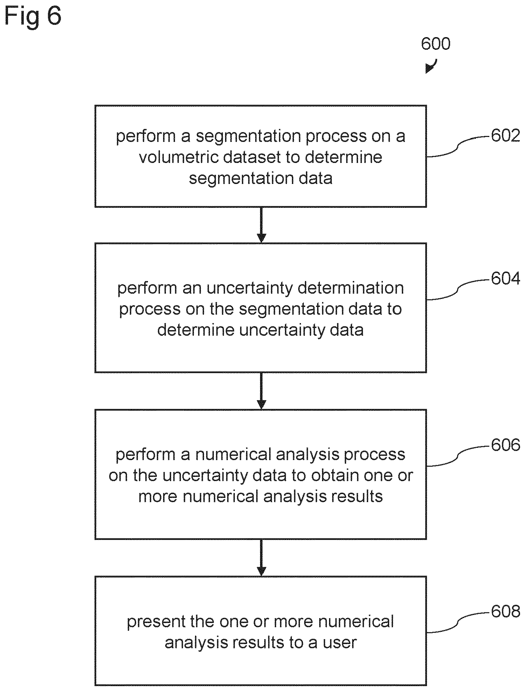

Figure 6 is a flow chart showing a computer-implemented method 600 for representing an uncertainty in a segmentation of a volumetric dataset representing a medical volume.

The method 600 comprises, at block 602 performing a segmentation process on a volumetric dataset to determine segmentation data indicative of an association between a given location in the volumetric dataset and a given one of one or more anatomical regions in the medical volume. Block 602 may comprise any of the features described above in relation to block 102 of the method 100.

At block 604, the method 600 comprises performing an uncertainty determination process on the segmentation data to determine uncertainty data indicative of an uncertainty in the association between the given location and the given one of the one or more anatomical regions. Block 604 may comprise any of the features described above in relation to block 104 of the method 100.

At block 606, the method 600 comprises performing a numerical analysis process on the uncertainty data to obtain one or more numerical analysis results.

For example, the uncertainty values for all of the voxels in the volumetric dataset may be summed to obtain a measure of a total uncertainty of the associations indicated by the segmentation data. This total uncertainty may, for example, be a sum of the maximum variances of each of the voxels in the dataset. In another example, the total uncertainty may be a sum across each of the voxels of the variances relating to a particular anatomical region, for example, the bladder. Another example measure which may be computed is the maximum uncertainty among the uncertainties of all of the voxels. A further example measure which may be computed is the total number of voxels for which the uncertainty exceeds a particular threshold. Such measures may provide useful information to allow a user to understand whether, in general, the segmentation result is highly certain or uncertain. With this understanding, the user may decide how to proceed in terms of reviewing the segmentation result in further detail.

At block 608, the method 600 comprises presenting the one or more numerical analysis results to a user.

In some examples, the results of the numerical analysis may be presented graphically to a user. For example, a traffic light display system may indicate to a user a degree to which manual review of the segmentation result may be required, based on the results of the numerical analysis. For example, if the results of the numerical analysis indicate that the uncertainty in the segmentation result is very low, then it may be indicated that no review is required, for example, by presenting a green traffic light graphic to the user. If the results of the numerical analysis indicate that the segmentation result is somewhat uncertain, then it may be indicated to the user that some manual review may be required, for example, by presenting an amber traffic light graphic to the user. If the results of the numerical analysis indicate that the uncertainty in the segmentation result is high, then it may be indicated that manual review is required, for example, by presenting a red traffic light graphic to the user.

The determination of which of these indications to present to the user may, for example, be based on applying one or more thresholds to the total uncertainty and/or the maximum uncertainty. In other examples, a combination of such results of the numerical analysis may be used to make the determination of the degree to which review manual may be required. In some examples, the determination may be made on a per anatomical region basis. For example, in the above-described examples of the pelvic region, numerical analysis of the uncertainty data may be performed separately for each of the left femur, right femur, and the bladder. The results of the numerical analysis for each of these anatomical regions may be presented to the user separately, for example, via separate respective traffic light graphics.

Such examples allow the uncertainty information to be presented to the user in a simple manner to avoid overloading the display with information for the user. This may also allow the user to clearly identify the degree to which review is required in each case and to minimize the risk that the user fails to recognize information indicating that the segmentation result should be reviewed. In some examples, the numerical analysis e.g. in the form of one or more traffic light graphics, may be displayed to the user without a full 3D visualization of the volumetric dataset and the uncertainty data. In some examples, a minimal representation of the results of the numerical analysis may be initially displayed to the user, with further detail of the results of the numerical analysis available on demand. Further, the 3D visualization of the volumetric dataset and the uncertainty data may also only be shown on demand, for example, if, responsive to the traffic light graphic indicating that the segmentation result needs review, the user requests the 3D visualization.

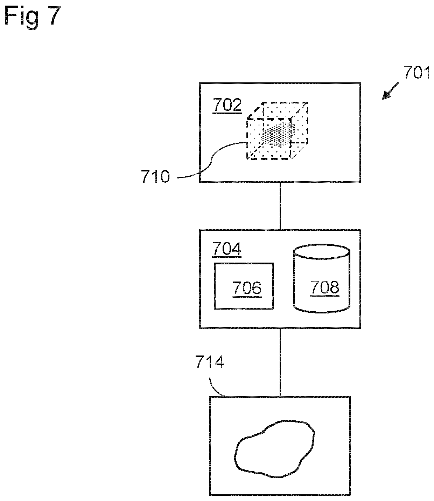

Figure 7 is a schematic drawing illustrating an example system 701 in which an example apparatus 704 may use methods described herein. The system 701 comprises a scanner 702, the apparatus 704, and a visualization unit 714. In examples, the system may comprise fewer components than or additional components to those illustrated in Figure 7. For example, the system 701 may comprise a computer network such as the internet.

The scanner 702 may be any scanner for generating a dataset comprising a volumetric dataset 710, which may, for example, be a medical volumetric dataset representing a portion of a patient. The scanner 702 may be a computed tomography (CT) scanner, a magnetic resonance imaging (MRI) scanner, a positron emission tomography (PET) scanner, an ultrasound scanner or the like. The scanner 702 is connected to the apparatus 704, for example via wired or wireless connection. The scanner 702 may be arranged to provide the volumetric dataset 710 to the apparatus 704.

The apparatus 704 comprises one or more processors 706 and a memory, in the form of one or more storages 708. The apparatus 704 may, for example, comprise a GPU. In this example, the apparatus 704 is arranged to perform a method according to examples described above of rendering an image of the volumetric dataset 710. The apparatus 704 may for example, comprise one or more processors for performing the various aspects of the method. For example, the apparatus 704 may operate one or more trained machine-learning models, for example, one or more neural networks, configured to perform the segmentation process on the volumetric dataset 710. The apparatus 704 may also comprise one or more modules for performing the uncertainty determination process on the segmentation data. The apparatus 704 may comprise a volume rendering module to perform the volume rendering process, e.g. by performing a physically based volume rendering on the volumetric dataset 710.

The storage 708 may comprise a machine-readable medium comprising a set of machine-readable instructions which when executed by the processor 706 cause the apparatus 704 to perform an example method described herein. The program may be stored on a computer readable medium which may be read by the apparatus 704 to thereby execute the program.

The apparatus 704 may be arranged to receive directly or indirectly or otherwise acquire from the scanner 702 the volumetric dataset 710.

The apparatus 704 may be arranged to transmit information, for example, values defining a rendering of the volumetric dataset 710, for example, a color value for each pixel in an image plane, to a visualization unit 714. The transmission may be direct or indirect, for example via a wired connection, a wireless connection, or via the internet.

The visualization unit 714 may comprise visualization software for displaying a two-dimensional projection of the volumetric dataset 710 produced by the apparatus 704. The visualization unit 714 may comprise a display screen, and one or more graphics hardware or software components. In some examples, the visualization unit 714 may be or comprise a mobile device. In some examples the visualization unit 714 may comprise a virtual reality or augmented reality device. The visualization unit 714 may in some examples display a stereo image.

While in certain examples, the segmentation volume and uncertainty volume is at a same resolution as the volumetric dataset, in other examples, the segmentation volume and/or the uncertainty volume may be at a different resolution to the volumetric dataset. For example, the segmentation volume might be at half the resolution of the volumetric dataset. In such an example, each segmentation voxel may be used to classify eight voxels of the volumetric dataset.

Although in some examples described above, the uncertainty volume is formed of a single respective overall uncertainty value for each voxel, in other examples, the uncertainty volume may comprise multiple uncertainty values for each voxel. For example, the uncertainty values relating to each of the anatomical regions of interest may all be stored in the uncertainty volume to be accessed when needed. In such an example, the manner in which the uncertainty value for a given location for use in the volume rendering process is determined may be dependent on the desired type of visualization, for example, as indicated by a user. For example, where a visualization of the segmentation result and related uncertainty for a given anatomical region is of interest, the uncertainty values from the uncertainty volume relating to that anatomical region may be sampled from the uncertainty volume. On the other hand, where it is desired to visualize the segmentation results for multiple anatomical regions of interest, the maximum uncertainty value for each voxel may be sampled from the uncertainty volume. Typically, the maximum uncertainty value for a given voxel will be the uncertainty value of the anatomical region with the highest determined probability for the given voxel. Accordingly, areas where the segmentation algorithm was least certain overall about the segmentation result can be easily identified in the visualization while viewing simultaneously the segmentation results for all of the anatomical regions of interest.

While in certain examples described herein, the volume rendering process is a path tracing process, in other examples, any suitable volume rendering process may be used to render the image of the medical volume including the visualization of the uncertainty data. For example, a direct volume rendering process, such as a ray casting process, may be used.

While certain examples described herein relate to visualizing the uncertainty in a segmentation result of organs at risk, similar principles can be applied to visualize the uncertainty of a segmentation of other anatomical regions. For example, in the context of RT, the segmentation process may be configured to identify a target of RT, such as a tumor region, for example the gross tumor volume (GTV), clinical target volume (CTV) or planning target volume (PTV) and the uncertainty data may relate to the uncertainty in this segmentation. In such examples, the uncertainty data may highlight the margins of the target that are poorly defined, or which are driven by clinical knowledge, such as guidelines, outside of the information obtained by the relevant scanning modality by which the volumetric dataset has been obtained.

The above embodiments are to be understood as illustrative examples of the invention. Other embodiments are envisaged. It is to be understood that any feature described in relation to any one embodiment may be used alone, or in combination with other features described, and may also be used in combination with one or more features of any other of the embodiments, or any combination of any other of the embodiments. Furthermore, equivalents and modifications not described above may also be employed without departing from the scope of the invention, which is defined in the accompanying claims.

Independent of the grammatical term usage, individuals with male, female or other gender identities are included within the term.

**Claims**

**1.** A computer-implemented method (100) for rendering a volumetric dataset (200) representing a medical volume, the method comprising:

performing (102) a segmentation process on a volumetric dataset (200) to determine segmentation data indicative of an association between a given location in the volumetric dataset (200) and a given one of one or more anatomical regions in the medical volume;

performing (104) an uncertainty determination process on the segmentation data to determine uncertainty data indicative of an uncertainty in the association between the given location and the given one of the one or more anatomical regions; and

performing (106) a volume rendering process on the volumetric dataset (200), using the uncertainty data, to render an image of the medical volume, including determining a visual characteristic of the image based on the uncertainty in the association between the given location and the given one of the one or more anatomical regions.

2. The method (100) of claim 1, wherein the volume rendering process comprises:
determining visual parameter data for a given position in the image based on one or more respective visual parameter values for one or more sample points in the volumetric dataset (200), and wherein the visual parameter value for a given sample point of the one or more sample points is determined based on:
an uncertainty value at the given sample point derived from the uncertainty data.

3. The method (100) of claim 2, wherein the visual parameter value for the given sample point is determined based on one or more of:

a volume value at the given sample point derived from the volumetric dataset (200); and

a segmentation value at the given sample point derived from the segmentation data.

4. The method (100) of claim 2 or claim 3, wherein the visual parameter value for the given sample point is determined by applying one or more transfer functions to one or more of:

the uncertainty value at the given sample point;

the or a volume value at the given sample point; and

the or a segmentation value at the given sample point.

5. The method (100) of claim 4, wherein the visual parameter value for the given sample point is determined by combining:

a color and/or an opacity value obtained by applying an uncertainty transfer function to the uncertainty value; with one or more respective color and/or opacity values obtained by applying respective transfer functions to one or more of the volume value at the given sample point and the segmentation value at the given sample point.

6. The method (100) of any preceding claim, wherein the volume rendering process is a physically based volume rendering process.

7. The method (100) of any preceding claim, wherein the segmentation process comprises applying a trained machine-learning model to the volumetric dataset (200) and obtaining the segmentation data as an output of the trained machine-learning model.

8. The method (100) of any preceding claim, wherein the uncertainty determination process comprises determining the uncertainty data by performing a statistical calculation on the segmentation data.

9. The method (100) of any preceding claim, wherein, for the given location in the volumetric dataset (200):

the segmentation data comprises first data including one or more respective probability values indicative of respective likelihoods that the given location corresponds to each of the one or more anatomical regions;

the uncertainty data comprises second data including one or more uncertainty values indicative of respective uncertainties in the respective probability values of the first data; and:

wherein the segmentation process comprises determining the association between the given location and the given one of the one or more anatomical regions based on the respective probability values of the first data; and/or

wherein the uncertainty determination process comprises determining the uncertainty in the association between the given location and the given one of the one or more anatomical regions based on the uncertainty values of the second data.

10. The method (100) of claim 9, wherein determining the uncertainty in the association between the given location and the given one of the one or more anatomical regions based on the uncertainty values of the second data comprises determining the largest of the uncertainty values of the second data.

11. The method (100) of any preceding claim, wherein the method comprises:
utilizing the image of the medical volume in a radiation therapy planning process.

12. A computer-implemented method (600) for representing an uncertainty in a segmentation of a volumetric dataset (200) representing a medical volume, the method comprising:

performing (602) a segmentation process on a volumetric dataset (200) to determine segmentation data indicative of an association between a given location in the volumetric dataset (200) and a given one of one or more anatomical regions in the medical volume;
performing (604) an uncertainty determination process on the segmentation data to determine uncertainty data indicative of an uncertainty in the association between the given location and the given one of the one or more anatomical regions;
performing (606) a numerical analysis process on the uncertainty data to obtain one or more numerical analysis results; and
presenting (608) the one or more numerical analysis results to a user.

13. The method (600) of claim 12, wherein presenting the one or more numerical analysis results to a user comprises presenting a graphical representation of the one or more numerical analysis results to the user.

14. A computer program or a computer-readable medium comprising instructions which, when executed by a computer, cause the computer to carry out the method according to any of claims 1 to 13.

15. Apparatus (704) comprising a processor (706) configured to perform the method according to any of claims 1 to 13.

**Amended claims in accordance with Rule 137(2) EPC.**

1. A computer-implemented method (100) for rendering a volumetric dataset (200) representing a medical volume, the method comprising:

performing (102) a segmentation process on a volumetric dataset (200) to determine segmentation data indicative of an association between a given location in the volumetric dataset (200) and a given one of one or more anatomical regions in the medical volume;
performing (104) an uncertainty determination process on the segmentation data to determine uncertainty data indicative of an uncertainty in the association between the given location and the given one of the one or more anatomical regions; and **characterised in that** the method comprises:

performing (106) a volume rendering process on the volumetric dataset (200), using the uncertainty data, to render an image of the medical volume, including determining a visual characteristic of the image based on the uncertainty in the association between the given location and the given one of the one or more anatomical regions;
wherein the volume rendering process comprises:
determining visual parameter data for a given position in the image based on one or more respective visual parameter values for one or more sample points in the volumetric dataset (200), and wherein the visual parameter value for a given sample point of the one or more sample points is determined based on:
an uncertainty value at the given sample point derived from the uncertainty data.

2. The method (100) of claim 1, wherein the visual parameter value for the given sample point is determined based on one or more of:

a volume value at the given sample point derived from the volumetric dataset (200); and

a segmentation value at the given sample point derived from the segmentation data.

3. The method (100) of claim 1 or claim 2, wherein the visual parameter value for the given sample point is determined by applying one or more transfer functions to one or more of:

the uncertainty value at the given sample point;
the or a volume value at the given sample point; and
the or a segmentation value at the given sample point.

4. The method (100) of claim 3, wherein the visual parameter value for the given sample point is determined by combining:

a color and/or an opacity value obtained by applying an uncertainty transfer function to the uncertainty value; with one or more respective color and/or opacity values obtained by applying respective transfer functions to one or more of the volume value at the given sample point and the segmentation value at the given sample point.

5. The method (100) of any preceding claim, wherein the volume rendering process is a physically based volume rendering process.

6. The method (100) of any preceding claim, wherein the segmentation process comprises applying a trained machine-learning model to the volumetric dataset (200) and obtaining the segmentation data as an output of the trained machine-learning model.

7. The method (100) of any preceding claim, wherein the uncertainty determination process comprises determining the uncertainty data by performing a statistical calculation on the segmentation data.

8. The method (100) of any preceding claim, wherein, for the given location in the volumetric dataset (200):

the segmentation data comprises first data including one or more respective probability values indicative of respective likelihoods that the given location corresponds to each of the one or more anatomical regions;
the uncertainty data comprises second data including one or more uncertainty values indicative of respective uncertainties in the respective probability values of the first data; and:

wherein the segmentation process comprises determining the association between the given location and the given one of the one or more anatomical regions based on the respective probability values of the first data; and/or
wherein the uncertainty determination process comprises determining the uncertainty in the association between the given location and the given one of the one or more anatomical regions based on the uncertainty values of the second data.

9. The method (100) of claim 8, wherein determining the uncertainty in the association between the given location and the given one of the one or more anatomical regions based on the uncertainty values of the second data comprises determining the largest of the uncertainty values of the second data.

10. The method (100) of any preceding claim, wherein the method comprises:
utilizing the image of the medical volume in a radiation therapy planning process.

11. A computer-implemented method (600) for representing an uncertainty in a segmentation of a volumetric dataset (200) representing a medical volume, the method comprising:

performing (602) a segmentation process on a volumetric dataset (200) to determine segmentation data indicative of an association between a given location in the volumetric dataset (200) and a given one of one or more anatomical regions in the medical volume;
performing (604) an uncertainty determination process on the segmentation data to determine uncertainty data indicative of an uncertainty in the association between the given location and the given one of the one or more anatomical regions;
performing (606) a numerical analysis process on the uncertainty data to obtain one or more numerical analysis results; and

presenting (608) the one or more numerical analysis results to a user.

12. The method (600) of claim 11, wherein presenting the one or more numerical analysis results to a user comprises presenting a graphical representation of the one or more numerical analysis results to the user.

13. A computer program or a computer-readable medium comprising instructions which, when executed by a computer, cause the computer to carry out the method according to any of claims 1 to 12.

14. Apparatus (704) comprising a processor (706) configured to perform the method according to any of claims 1 to 12.

EP 4 645 218 A1

Fig 1

100

```
┌─────────────────────────────────────┐
│ perform a segmentation process on a  │── 102
│   volumetric dataset to determine     │
│        segmentation data              │
└─────────────────────────────────────┘
                   │
                   ▼
┌─────────────────────────────────────┐
│ perform an uncertainty determination │── 104
│   process on the segmentation data to │
│      determine uncertainty data       │
└─────────────────────────────────────┘
                   │
                   ▼
┌─────────────────────────────────────┐
│ perform a volume rendering process on │── 106
│     the volumetric dataset, using the │
│   uncertainty data, to render an image of │
│          the medical volume           │
└─────────────────────────────────────┘
```

Fig 2

EP 4 645 218 A1

## Fig 3

18

Fig 4

Fig 5

Fig 6

600

perform a segmentation process on a
volumetric dataset to determine
segmentation data —602

perform an uncertainty determination
process on the segmentation data to
determine uncertainty data —604

perform a numerical analysis process
on the uncertainty data to obtain one or
more numerical analysis results —606

present the one or more numerical
analysis results to a user —608

Fig 7

Europäisches
Patentamt

European
Patent Office

Office européen
des brevets

# EUROPEAN SEARCH REPORT

**Application Number**

EP 24 17 3486

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/122248 A1 (KONINKLIJKE PHILIPS NV [NL]) 24 June 2021 (2021-06-24) * page 12, line 20 - page 15, line 13; figures 2-4 * ----- | 1-15 | INV. G06T7/00 G06T7/11 |

**TECHNICAL FIELDS SEARCHED (IPC)**

G06T
G06V

The present search report has been drawn up for all claims

| Place of search | Date of completion of the search | Examiner |
|---|---|---|
| The Hague | 8 October 2024 | Grigorescu, Simona |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 17 3486

This annex lists the patent family members relating to the patent documents cited in the above-mentioned European search report.
The members are as contained in the European Patent Office EDP file on
The European Patent Office is in no way liable for these particulars which are merely given for the purpose of information.

08-10-2024

| Patent document cited in search report | Publication date | Patent family member(s) | | Publication date |
|---|---|---|---|---|
| WO 2021122248 A1 | 24-06-2021 | CN | 114846510 A | 02-08-2022 |
| | | EP | 3848892 A1 | 14-07-2021 |
| | | EP | 4078511 A1 | 26-10-2022 |
| | | US | 2023360225 A1 | 09-11-2023 |
| | | WO | 2021122248 A1 | 24-06-2021 |

EPO FORM P0459

For more details about this annex : see Official Journal of the European Patent Office, No. 12/82